# EUROPEAN PATENT SPECIFICATION

(11) **EP 3 035 777 B1**
(45) Date of publication and mention of the grant of the patent: **08.02.2017**
(21) Application number: 15197444.1
(22) Date of filing: 02.12.2015
(51) Int. Cl.: H05H 7/12, A61N 5/00, G21K 1/00

(54) **ROTATING ENERGY DEGRADER**
ROTIERENDER ENERGIEABSTUFER
DISPOSITIF TOURNANT DE DÉGRADATION D'ÉNERGIE

(30) Priority: 16.12.2014 EP 14198364
(43) Date of publication of application: 22.06.2016
(73) Proprietor: ION BEAM APPLICATIONS S.A., 1348 Louvain-la-Neuve (BE)
(72) Inventor: CLAEREBOUDT, Yves, 1348 LOUVAIN-LA-NEUVE (BE); DEBATTY, Alexandre, 1348 LOUVAIN-LA-NEUVE (BE); GERARD, Nicolas, 1348 LOUVAIN-LA-NEUVE (BE)
(74) Representative: De Groote, Christophe

(56) References cited:
- EP-A1- 1 541 194
- US-A- 6 034 377
- US-A1- 2004 200 983
- US-A1- 2012 267 544
- US-B1- 6 433 336

## Description

### Field of the invention

The invention relates to the field of charged particle accelerators, such as proton or carbon ion accelerators for example, and more particularly to a rotating energy degrader for attenuating the energy of a charged particle beam extracted from such a particle accelerator.

The invention also relates to a particle therapy system comprising a particle accelerator and a rotating energy degrader for attenuating the energy of a charged particle beam extracted from the particle accelerator.

### Description of prior art

Certain applications involving the use of beams of charged particles require the energy of these particles to be varied. This is for example the case in particle therapy applications, where the energy of the charged particles determines the depth of penetration of these particles into a body to be treated by such therapy. Fixed-energy particle accelerators, such as cyclotrons for instance, are not themselves adapted to vary the energy of the particle beam which they produce, and therefore require an additional device to vary this energy. Variable-energy particle accelerators, such as synchrotrons for instance, are themselves adapted to vary the energy of the particle beam which they produce, but it may nevertheless be desirable to further vary the energy after the particles have been extracted from a synchrotron.
Devices for varying the energy of a particle beam extracted from a particle accelerator are generally called energy degraders. An energy degrader comprises therefore one or more blocks of matter which are placed across the path of the particle beam after its extraction from the particle accelerator. According to a well-known principle, a charged particle passing through the thickness of such a block of matter undergoes a decrease in its energy by an amount which is, for particles of a given type, a function of the intrinsic characteristics of the material passed through and of said thickness.

A known rotating energy degrader is for example disclosed in US6433336. It comprises a single block of energy degrading material which has the shape of a plain helical staircase with discrete flat steps and which is placed across the path of the particle beam. The particle beam enters the degrader perpendicularly to a step of the staircase and exits the degrader at the opposite side, which attenuates the energy of the beam according to the thickness of the degrader at said step. After having rotated the staircase by a given angle around its axis, the particle beam will enter the degrader perpendicularly to another step and exit the degrader at the opposite side, which will attenuate the energy of the beam by a different amount according to the thickness of the degrader at said other step. The energy attenuation can thus varied by changing the angular position of the degrader with respect to the particle beam.

A drawback of these known energy degraders is that they must have a large diameter in order to have steps of sufficiently small height ("H" in Fig.1c of US6433336) to obtain the resolution in energy variation which is required for particle therapy applications for example.
As a consequence, these degraders have a large moment of inertia, so that it is difficult to make them rotate quickly and/or with high accuracy with respect to the particle beam. Some recent applications require however to be able to change the energy of the particle beam very quickly, such as in a few tens of milliseconds for instance, and/or with high accuracy. This is for example the case with particle therapy systems, where a target, such as a tumour for example, is to be irradiated layer by layer with the particle beam, these layers being at different depths into the body of the patient. In such cases, it is desirable to be able to change the energy of the particle beam very quickly and/or very accurately when the system passes from the irradiation of one layer to the irradiation of another layer.
Another drawback of the large diameter of the known degraders is that they require large quantities of expensive energy degrading material, which make them quite costly. A further drawback of their large diameter is that they are cumbersome and occupy lots of space, especially footprint space.

Another known rotating energy degrader is for example disclosed by R.E. Berg in "Rotating wedge cyclotron beam degrader" (Proceedings of the 7th International conference on cyclotrons and their applications; Zürich, Switzerland, 19-22 August 1975; pp.315-316).
It comprises a "comma"-shaped block of matter which is rotatably movable around an axis which is perpendicular to the "comma". The beam crosses the comma in a direction which is perpendicular to the rotation axis and hence enters into the "comma" at an outer curved side of the "comma" and exits out of the "comma" at an inner curved side of the "comma", or vice-versa. The energy attenuation is varied by changing the angular position of the "comma" with respect to the particle beam.
These energy degraders would also require a large diameter, particularly if they would be used for particle therapy applications, and therefore present similar drawbacks as the one disclosed in US6433336, namely a high moment of inertia, high cost and high occupied volume.

### Summary of the invention

It is an object of the invention to address the drawbacks of the known energy degraders. It is a particular object of the invention to provide an energy degrader which is adapted to vary the energy of a particle beam more quickly and/or with higher accuracy than the known degraders.

A typical beam energy at an input of an energy degrader according to the invention is in the MeV range, such as in the range of 150 MeV to 300 MeV for example, and a typical desired beam energy at an output of an energy degrader according to the invention is also in the MeV range, such as in the range of 50 MeV to 230 MeV for an upstream energy of 230 MeV for example.

The invention is defined by the independent claims. The dependent claims define advantageous embodiments.

According to the invention, there is provided an energy degrader for attenuating the energy of a charged particle beam extracted from a particle accelerator, according to claim 1.

It is to be noted that a helical surface may have a close-up appearance of a helical staircase with very small steps, for example in case an energy attenuation member is made with a 3D printer, but that it is still to be considered as a continuous helical surface in case a minimum run (tread depth) of its steps is smaller than a minimum average beam diameter at a level where the beam crosses the helical surface (for example a minimum run of its steps which is smaller than 8 mm in case of an average beam diameter ranging between 8 mm and 30 mm when crossing the helical surface).

As will also appear hereafter from the figures showing embodiments of the invention, the degrader is hence geometrically arranged so that the respective entry and exit faces of the energy attention members are disposed in the following (continuous or discontinuous) sequence with respect to the path of a charged particle beam crossing it:
- the beam entry face of the first energy attenuation member,
- the beam exit face of the first energy attenuation member,
- the beam entry face of the second energy attenuation member, and
- the beam exit face of the second energy attenuation member.

By "discontinuous sequence", it must be understood that additional attenuation material may be present in-between the beam exit and entry faces of respectively the first and second energy attenuation members, such as a flat material plate for example.

Thanks to the presence of the two energy attenuation members having their two facing and continuous helical surfaces with the same handedness, the diameter of the degrader can be made smaller than with known rotating degraders, yet providing for a good resolution in energy variation and for a limited statistical energy spread of the particles at the output of the degrader. With a smaller diameter, and hence a smaller moment of inertia, it will be possible to rotate the energy attenuation member(s) more quickly and therefore the energy of the particle beam can be varied more quickly. Such a degrader also requires less space.

Preferably, the beam entry face of the first energy attenuation member is perpendicular to the first axis and in the beam exit face of the second energy attenuation member is perpendicular to the second axis. This allows to limit even more the statistical energy spread of the particles at the output of the degrader.

Preferably, the drive assembly comprises a first motor for rotating the first energy attenuation member around the first axis and a second motor for rotating the second energy attenuation member around the second axis. Compared to a configuration wherein the first energy attenuation member would be fixed and the second energy attenuation member would be mobile in rotation, such a preferred configuration allows, for a given/desired energy attenuation, to position the first and second energy attenuation members independently from each other with respect to the particle beam, for example according to the characteristics of the beam optics at the beam entry and exit faces. It presents the further advantage to enable a faster and more accurate variation of the energy of the particle beam.

Preferably, the first and the second helical surfaces are cylindrical helical surfaces. More preferably, the first and the second helical surfaces have the same radius and the first axis is the same as the second axis. Even more preferably, the first and the second helical surfaces have the same pitch. Even more preferably, the first and second energy attenuation members are identical in shape and size. This allows easy and cheaper manufacturing of the degrader.

Alternatively, the radius of the first helical surface is smaller than the radius of the second helical surface, the pitch of the first helical surface is smaller than the pitch of the second helical surface, and the first axis is different from and parallel to the second axis. With such alternative, the first energy attenuation member will have an even smaller diameter and hence will be able to move even faster.

According to the invention, there is also provided a particle therapy system comprising a particle accelerator and an energy degrader according to the invention, said energy degrader being positioned and oriented with respect to a particle beam extracted from the particle accelerator, in such a way that the particle beam enters the energy degrader at the beam entry face of the first energy attenuation member and in such a way that said particle beam exits the energy degrader at the beam exit face of the second energy attenuation member. In case the beam entry face of the first energy attenuation member is perpendicular to the first axis and the beam exit face of the second energy attenuation member is perpendicular to the second axis, the energy degrader is preferably positioned and oriented with respect to a particle beam extracted from the particle accelerator, in such a way that the particle beam enters the energy degrader perpendicularly to the beam entry face of the first energy attenuation member.

Preferably, the particle accelerator is a fixed-energy accelerator, more preferably a cyclotron, even more preferably a synchrocyclotron.

### Short description of the drawings

These and further aspects of the invention will be explained in greater detail by way of example and with reference to the accompanying drawings in which:
- Fig.1: schematically shows a front view of an exemplary energy degrader according to the invention;
- Fig.2: schematically shows a top view of the energy degrader of Fig.1;
- Fig.3: schematically shows a partial sectional view of the energy degrader of Fig.1 at a high energy attention level;
- Fig.4: schematically shows a partial sectional view of the energy degrader of Fig.1 at a low energy attention level;
- Fig.5: schematically shows a front view of another exemplary energy degrader according to the invention;
- Fig.6: schematically shows a top view of the energy degrader of Fig.5;
- Fig.7: schematically shows a front view of a part of another exemplary energy degrader according to the invention;
- Figs.8a, 8b, 8c: schematically show a front view of the energy degrader of Fig. 7 at various attenuation levels;
- Fig.9: schematically shows a particle therapy system comprising a particle accelerator and an energy degrader according to the invention.

The drawings of the figures are neither drawn to scale nor proportioned. Generally, similar or identical components are denoted by the same reference numerals in the figures.

### Detailed description of embodiments of the invention

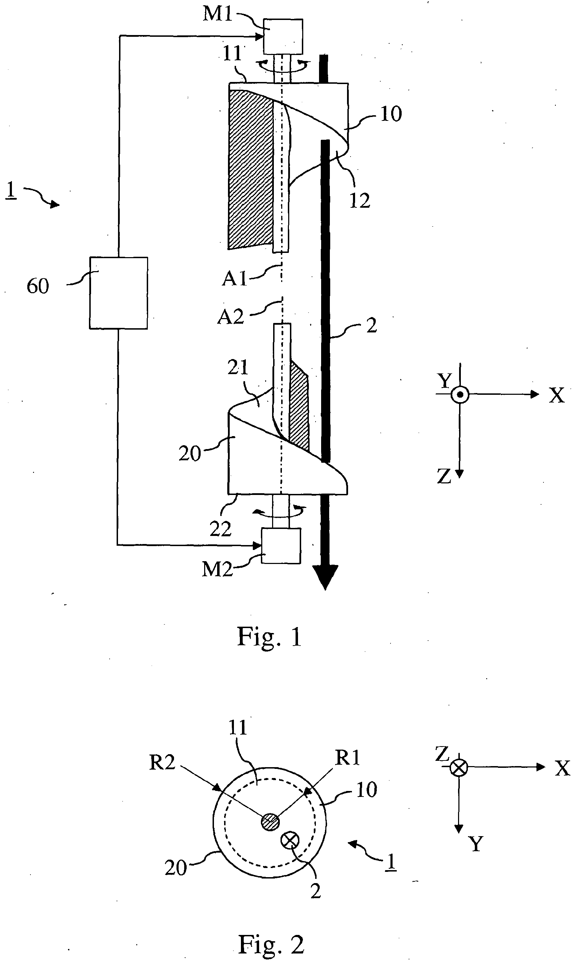

Fig.1 schematically shows a front view of an exemplary energy degrader (1) according to the invention, in an XYZ referential.

The energy degrader (1) comprises two disjoint energy attenuation members: a first energy attenuation member (10) and a second energy attenuation member (20).
The first energy attenuation member (10) presents a beam entry face (11) having the shape of an annulus or a disk (or a portion thereof), and it presents an opposed beam exit face (12) having the shape of a part of a first continuous helical surface having a first axis (A1).
The second energy attenuation member (20) presents a beam entry face (21) having the shape of part of a second continuous helical surface having a second axis (A2) which is parallel to or coincident with the first axis (A1), and it presents an opposed beam exit face (22) having the shape of an annulus or a disk (or a portion thereof).

It is to be noted that a helical surface may have a close-up appearance of a helical staircase with very small steps, for example in case an energy attenuation member is made with a 3D printer, but that it is still to be considered as a continuous helical surface in case a minimum run (tread depth) of its steps is smaller than a minimum average beam diameter at a level where the beam crosses the helical surface (for example a minimum run of its steps which is smaller than 8 mm in case of an average beam diameter ranging between 8 mm and 30 mm when crossing the helical surface).

As can be seen on Fig.1, the first and second energy attenuation members (10, 20) are positioned with respect to each other in such a way that the first and second helical surfaces are facing each other. The first and the second helical surfaces have the same handedness.
Preferably, the beam entry face (11) of the first energy attenuation member (10) is perpendicular to the first axis (A1) and the beam exit face (22) of the second energy attenuation member (20) is perpendicular to the second axis (A2). In such a preferred case, the beam entry face (11) of the first energy attenuation member (10) is of course parallel to the beam exit face (22) of the second energy attenuation member (20).

The energy degrader (1) further comprises a drive assembly which is operably connected to the first and the second energy attenuation members (10, 20). This drive assembly is configured for driving the first energy attenuation member (10) and/or the second energy attenuation member (20) into rotation around respectively the first axis (A1) and/or the second axis (A2), said first axis (A1) being parallel to or coincident with said second axis (A2).
The drive assembly may for example comprise a single motor as well as an optional transmission linking said single motor to the first energy attenuation member (10) so as to rotate the first energy attenuation member (10) around the first axis (A1), the second energy attenuation member (20) being fixed (not rotating).
Alternatively, the drive assembly may for example comprise a single motor as well as a transmission linking said single motor to respectively the first and the second energy attenuation members so as to rotate respectively the first and the second energy attenuation members, preferably in opposite directions (i.e. when the first energy attenuation member (10) is driven to rotate clockwise, the second energy attenuation member (20) will be driven to rotate anticlockwise and vice-versa).
Preferably, and as shown on Fig.1, the drive assembly comprises a first motor (M1) operably connected to the first energy attenuation member (10) for rotating the first energy attenuation member around the first axis (A1), and a second motor (M2) operably connected to the second energy attenuation member (20) for rotating the second energy attenuation member around the second axis (A2). The first and second motors may be stepper motors for example. Though not shown on Fig.1, the energy degrader (1) may further comprise (an) intermediary transmission(s) between the first and/or the second motors on the one hand and respectively the first and second energy attenuation members on the other hand, in order to adapt the speed and/or the torque applied by the motors to their corresponding energy attenuation members.

On Fig.1 is further shown a particle beam (2) when crossing the first and second energy attenuation members (10, 20). Given the geometry of these two attenuation members, it will be clear that an energy of an incoming beam will be more or less attenuated in function of the angular position(s) of the first and the second energy attenuation members with respect to the particle beam. A control unit (60), operably connected to the drive assembly, may be used to modify said angular positions, for example in function of energy attenuation settings received from a system using the energy degrader (1).

Fig.2 schematically shows a top view of the energy degrader (1) of Fig.1, whereon the annular shape of the entry face (11) of the first energy attenuation member (10) can be better seen.

Preferably, the first and the second helical surfaces are cylindrical helical surfaces, as can be seen from on the example of Figs. 1 and 2. Preferably, the first axis (A1) is the same as (coincident with) the second axis (A2). More preferably, the radius (R1) of the first helical surface is the same as the radius (R2) of the second helical surface. Even more preferably, the first and the second helical surfaces have the same pitch. Even more preferably, the first and second energy attenuation members (10, 20) are identical in shape and in size.

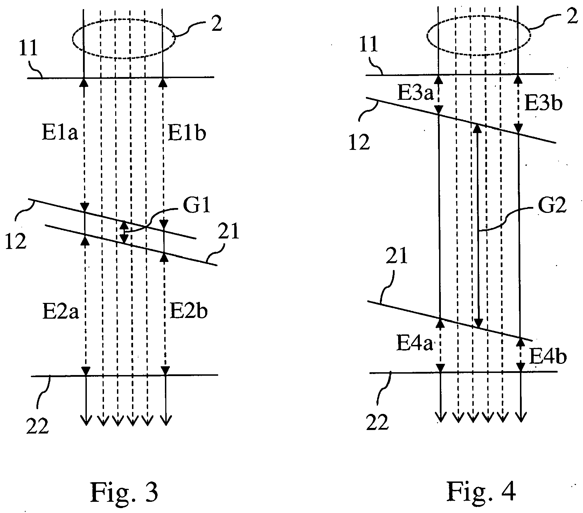

Fig.3 schematically shows a partial sectional and developed view of the energy degrader (1) of Fig.1 at a high energy attention level. In this exemplary representation, the first and second energy attenuation members have the same size and the same shape, which means that the first and second helical surfaces have the same radius, the same handedness and the same pitch. The particle beam (2) is here shown enlarged in order to more clearly see its sectional size. As can be seen on Fig.3, a particle of the leftmost part of the beam (2) will travel through thicknesses E1 a and E2a of the two energy attenuation members via a gap G1. A particle of the rightmost part of the beam (2) will travel through two thicknesses E1 b and E2b of the two energy attenuation members via the same gap G1. The gap G1 may for example be an air gap or a vacuum gap. A total attenuation of the energy of a particle may be estimated as the sum of the energy attenuations provided by the first and the second energy attenuation members along the path of the particle. In this exemplary configuration, we have that E1a+G1+E2a = E1b+G1+E2b, so that it will be understood that the energy of these two particles will be attenuated by approximately the same amount. The same holds for the other particles of the beam (2).

Fig.4 schematically shows a partial sectional and developed view of the energy degrader (1) of Fig.1 at a low energy attention level. This configuration may be obtained by rotating the first energy attenuation member (10) by a certain angle in the appropriate direction (in order to reduce the thicknesses E1 a and E1 b) and/or by rotating the second energy attenuation member (20) by a certain angle in the opposite direction (in order to reduce the thicknesses E2a and E2b). As can be seen on Fig.4, a particle of the leftmost part of the beam (2) will travel through thicknesses E3a and E4a of the two energy attenuation members via a gap G2. A particle of the rightmost part of the beam (2) will travel through two thicknesses E3b and E4b of the two energy attenuation members via the same gap G2. It will therefore be understood that the energy of these two particles will be attenuated by approximately the same amount. The same holds for the other particles of the beam (2). In this example, the gap G2 is larger than the gap G1, which is not that much of a problem because the attenuation level is low and therefore the dispersion of the beam is smaller than with the higher attenuation shown in Fig. 3. It is known that the beam size is more critical at the higher attenuation levels, and, as shown in Fig.3, the gap G1 can be made small there.

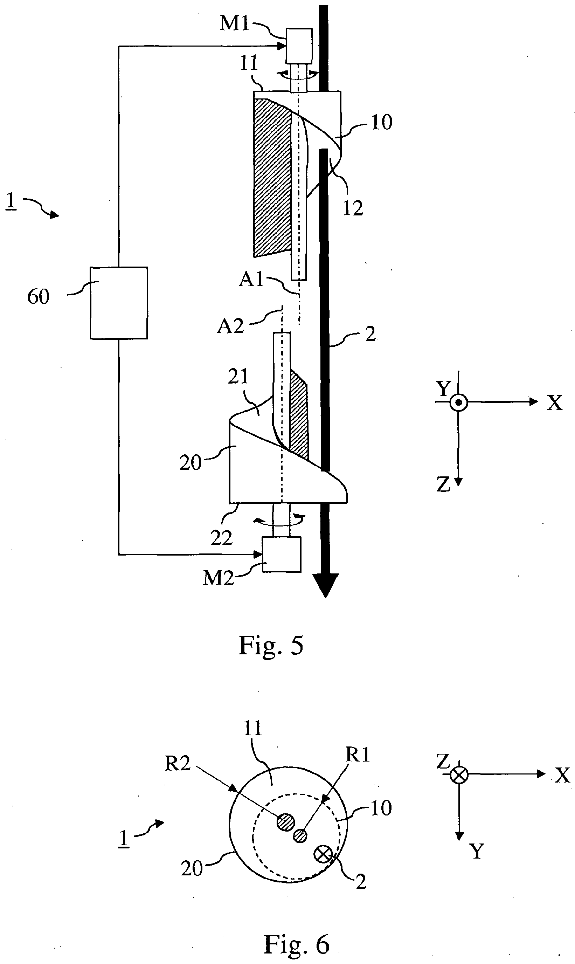

Fig. 5 schematically shows a front view of another exemplary energy degrader (1) according to the invention, in an XYZ referential. It is similar to the degrader of Fig.1, except that, in this case, the radius (R1) of the first helical surface is smaller than the radius (R2) of the second helical surface, and that the first axis (A1) is different from and parallel to the second axis (A2). Preferably, R1 < 0,5.R2, more preferably, R1 < 0,2.R2, even more preferably R1 < 0,1.R2.

In order to have the same slope on both helical surfaces, the pitch of the first helical surface is preferably smaller than the pitch of the second helical surface. Fig.6 schematically shows a top view of the energy degrader (1) of Fig.5.

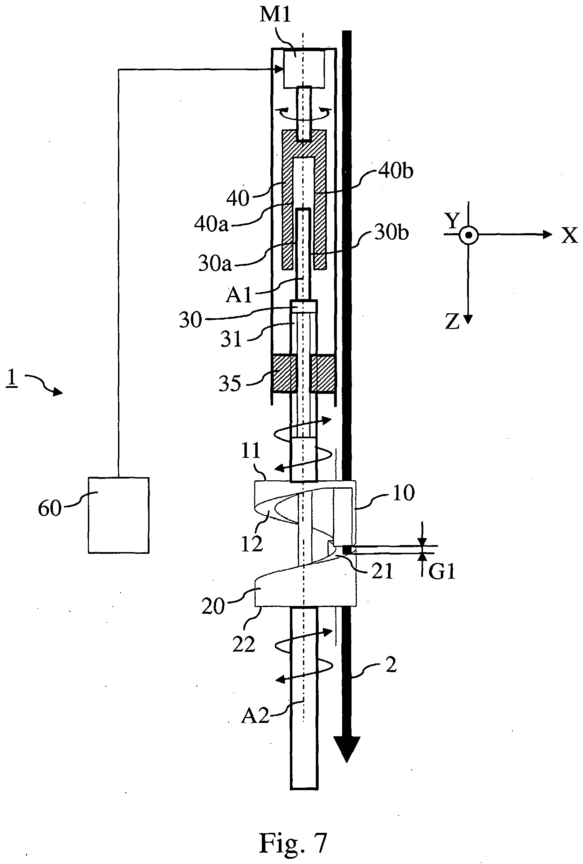

Fig.7 schematically shows a front view of a part of another exemplary energy degrader (1) according to the invention in an XYZ referential. It is similar to the degrader of Fig.1, except that, in this case, the stator of the first motor (M1) is attached to a guiding piece (35) comprising a threaded hole, and the first energy attenuation member (10) is attached to a shaft (30) comprising a threaded portion (31) passing through and cooperating with the threaded hole of the guiding piece (35).
The rotor of the first motor (M1) comprises a mechanical coupling (40) to the said shaft (30) for driving the shaft (30) into rotation while allowing an axial translation movement of the shaft (30). In this example, said mechanical coupling (40) comprises a "U"-shaped part having two flat inner portions (40a, 40b) slidingly engaging with respectively two flat external faces (30a, 30b) of a distal portion of the shaft (30).
The rotor of the first motor (M1), the threaded hole, the first helical surface of the first energy attenuation member (10) and the said shaft (30) are all coaxial in this example and have as axis the first axis (A1).
The pitch and the handedness of the threaded portion (31) of the shaft (30) (and hence also of the threaded hole of the guiding piece (35)) are the same as respectively the pitch and the handedness of the first helical surface.
It will therefore be understood that the drive assembly is adapted to move the first energy attenuation member (10) according to a helical movement around the first axis (A1), as shown by a helical double arrow on Fig. 7.

In this example, the rotor of the first motor (M1) is directly connected to the mechanical coupling (40). Preferably, the rotor of the first motor (M1) is connected to the mechanical coupling (40) via a speed reducer - such as a speed reduction gearbox for example - in order to increase the accuracy of the movement.

The second energy attenuation member (20) is preferably connected in the same way to the second motor (M2) (not shown on Fig. 7). The case being, it will be understood that the drive assembly is adapted to move the second energy attenuation member (20) according to a helical movement around the first axis (A1), as shown by a helical double arrow on Fig. 7. The control unit (60) is in such a case preferably configured to drive the first and second motors (M1, M2) synchronously, at the same speed and in opposite directions, and in such a way that when the first motor (M1) rotates the first energy attenuation member (10) of an angle α (alpha), the second motor (M2) rotates the second energy attenuation member (20) of an angle - α (minus alpha). With such a configuration, it becomes possible to place the first and second helical surfaces very close to each other and with a constant gap (G1) between them, whatever their angular position, i.e. whatever the level of energy attenuation. Preferably, the gap (G1) between the first and second helical surfaces is kept smaller than 5 cm, preferably smaller than 1 cm, preferably smaller than 5 mm, preferably smaller than 1 mm. Preferably, the first and second helical surfaces do not touch each other in order to avoid wear.
Preferably, the first and second helical surfaces each make a maximum turn of less than 360°, more preferably less than 270°, even more preferably less than or equal to 180° around their respective axis.

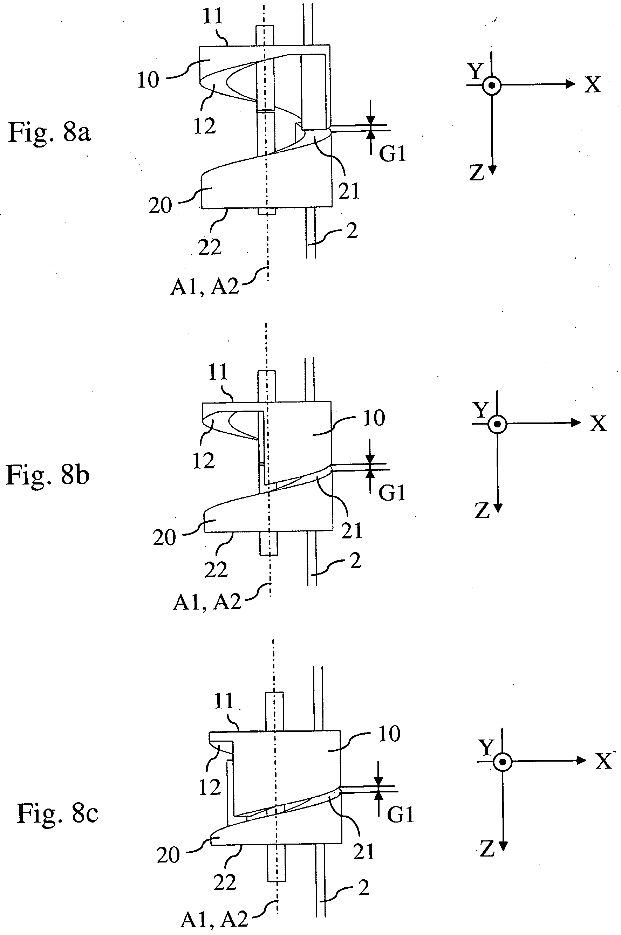

Figs.8a, 8b, 8c schematically show a front view of the energy degrader (1) of Fig. 7 at three different energy attenuation levels. On Fig 8a, the first and second energy attenuation members (10, 20) are angularly positioned by the drive assembly so that the particle beam (2) traverses a large thickness of material of both energy attenuation members, which results in a large energy attenuation. On Fig 8b, the first and second energy attenuation members (10, 20) have each been rotated by the drive assembly in opposite directions and by a same angle, so that the particle beam (2) traverses a smaller thickness of material compared to Fig.8a, which results in a smaller energy attenuation. On Fig 8c, the first and second energy attenuation members (10, 20) have each been further rotated by the drive assembly in opposite directions and by a same angle, so that the particle beam (2) traverses a smaller thickness of material compared to Fig.8b, which results in an even smaller energy attenuation.

Preferably, the first energy attenuation member (10) and/or the second energy attenuation member (20) are made of beryllium or carbon graphite. More preferably, the first energy attenuation member (10) is made of the same material as the second energy attenuation member (20).

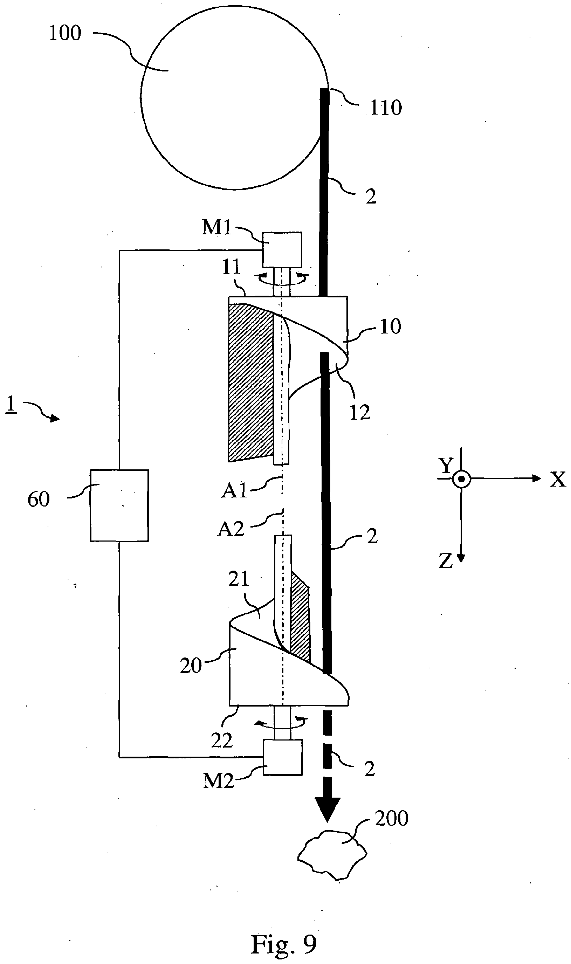

As schematically shown on Fig.9, the invention also concerns a particle therapy system configured for irradiating a target (200) with a charged particle beam (2) (50). Said particle therapy system comprises a particle accelerator (100) configured for generating and extracting a beam (2) of charged particles, such as a beam of protons or carbon ions for example, and an energy degrader (1) as described hereinabove for attenuating the energy of said charged particle beam (2) before it reaches the target (200).
The energy degrader (1) is positioned and oriented with respect to a particle beam (2) extracted from the particle accelerator (100), in such a way that the particle beam (2) enters the energy degrader (1) at the beam entry face (11) of the first energy attenuation member (10) and in such a way that said particle beam (2) exits the energy degrader (1) at the beam exit face (22) of the second energy attenuation member (20). Preferably, the beam entry face (11) of the first energy attenuation member (10) is parallel to beam exit face (22) of the second energy attenuation member (20) and it is perpendicular to the first axis (A1), the latter being parallel to or coincident with the second axis (A2). In this case, the energy degrader (1) is preferably positioned and oriented with respect to the particle beam (2), in such a way that the particle beam enters the energy degrader (1) perpendicularly to the beam entry face (11) of the first energy attenuation member (10), as shown on Fig. 9.

For the sake of clarity, Fig. 9 does not necessarily show all components of a particle therapy system, which are generally well known from the prior art, but only those components which are necessary to understand the present invention.

Preferably, the particle accelerator (100) is a fixed-energy accelerator, preferably a cyclotron, more preferably a synchrocyclotron.

Preferably, the particle accelerator (100) is configured for delivering at its output (110) a particle beam (2) whose maximal energy is comprised between 1 MeV and 500 MeV, preferably between 100 MeV and 300 MeV, more preferably between 200 MeV and 250 MeV.

In such a case, a typical desired beam energy at an output (22) of an energy degrader (1) according to the invention is also in the MeV range, such as in the range of 50 MeV to 230 MeV for an upstream energy of 230 MeV for example.

With these exemplary energies, which are common in particle therapy applications, one preferably has that:
- a minimal thickness (taken axially) of the first energy attenuation member (10) lies in the interval [1 mm, 100 mm], more preferably [1 mm, 50 mm], even more preferably [1 mm, 10 mm]. The same holds for the second energy attenuation member (20),
- a maximal thickness (taken axially) of the first energy attenuation member (10) lies in the interval [10 mm, 300 mm], more preferably [10 mm, 200 mm], even more preferably [10 mm, 100 mm]. The same holds for the second energy attenuation member (20), and
- a diameter of the first energy attenuation member (10) lies in the interval [10 mm, 300 mm], more preferably [10 mm, 200 mm], even more preferably [10 mm, 150 mm]. The same holds for the second energy attenuation member (20).

The invention may also be described as follows: an energy degrader (1) for attenuating the energy of a charged particle beam (2), comprising a first energy attenuation member (10) presenting a annular beam entry face and a helical beam exit face, a second energy attenuation member (20) presenting a helical beam entry face and an annular beam exit face, the first and second helical surfaces facing each other, and a drive assembly configured for rotating the first and/or the second energy attenuation members around respectively a first axis (A1) and a second axis (A2) which are parallel to each other.

The present invention has been described in terms of specific embodiments, which are illustrative of the invention and not to be construed as limiting. More generally, it will be appreciated by persons skilled in the art that the present invention is not limited by what has been particularly shown and/or described hereinabove.
Reference numerals in the claims do not limit their protective scope.
Use of the verbs "to comprise", "to include", "to be composed of", or any other variant, as well as their respective conjugations, does not exclude the presence of elements other than those stated.
Use of the article "a", "an" or "the" preceding an element does not exclude the presence of a plurality of such elements.

## Claims

1. An energy degrader (1) for attenuating the energy of a charged particle beam (2) extracted from a particle accelerator, said energy degrader (1) comprising:
- a first energy attenuation member (10) presenting a beam entry face (11) having the shape of an annulus or a disk, or a portion thereof, and a beam exit face (12) having the shape of a part of a first continuous helical surface having a first axis (A1),
- a second energy attenuation member (20) presenting a beam entry face (21) having the shape of part of a second continuous helical surface having a second axis (A2) and a beam exit face (22) having the shape of an annulus or a disk, or a portion thereof,
the first axis (A1) being parallel to or coincident with the second axis (A2), the first and second helical surfaces facing each other and having the same handedness,and
- a drive assembly, either operably connected to the first and to the second energy attenuation members and configured for rotating the first and the second energy attenuation members around respectively the first axis (A1) and the second axis (A2), or operably connected to the first energy attenuation member and configured for rotating the first energy attenuation member around the first axis (A1), the second energy attenuation member being fixed.

2. An energy degrader (1) according to claim 1, **characterized in that** the beam entry face (11) of the first energy attenuation member (10) is perpendicular to the first axis (A1) and **in that** the beam exit face (22) of the second energy attenuation member (20) is perpendicular to the second axis (A2).

3. An energy degrader (1) according to claim 1 or 2, **characterized in that** the drive assembly comprises a first motor (M1) for rotating the first energy attenuation member (10) around the first axis (A1) and a second motor (M2) for rotating the second energy attenuation member (20) around the second axis (A2).

4. An energy degrader (1) according to any of previous claims, **characterized in that** the first and the second helical surfaces are cylindrical helical surfaces.

5. An energy degrader (1) according to claim 4, **characterized in that** the first and the second helical surfaces have the same radius and **in that** the first axis (A1) is the same as the second axis (A2).

6. An energy degrader (1) according to claim 5, **characterized in that** the first and the second helical surfaces have the same pitch.

7. An energy degrader (1) according to claim 6, **characterized in that** the drive assembly is adapted to move the first and/or the second energy attenuation members according to a helical movement around the first axis (A1).

8. An energy degrader (1) according to claim 6 or 7, **characterized in that** the first and second energy attenuation members are identical in shape and size.

9. An energy degrader (1) according to claim 4, **characterized in that** the radius (R1) of the first helical surface is smaller than the radius (R2) of the second helical surface, **in that** the pitch of the first helical surface is smaller than the pitch of the second helical surface, and **in that** the first axis (A1) is different from and parallel to the second axis (A2).

10. An energy degrader (1) according to any of previous claims, **characterized in that** the first energy attenuation member (10) and/or the second energy attenuation member (20) are made of beryllium or carbon graphite.

11. A particle therapy system comprising a particle accelerator (100) and an energy degrader (1) according to any of previous claims, said energy degrader (1) being positioned and oriented with respect to a particle beam (2) extracted from the particle accelerator (100), in such a way that the extracted particle beam (2) enters the energy degrader (1) at the beam entry face (11) of the first energy attenuation member (10) and in such a way that said extracted particle beam (2) exits the energy degrader (1) at the beam exit face (22) of the second energy attenuation member (20).

12. A particle therapy system according to claim 11, **characterized in that** the particle accelerator (100) is a fixed-energy accelerator, preferably a cyclotron, more preferably a synchrocyclotron.

13. A particle therapy system according any of claims 11 for 12, **characterized in that** the particle accelerator (100) is configured for delivering at its output (110) a particles beam (2) whose maximal energy is comprised between 1 MeV and 500 MeV, preferably between 100 MeV and 300 MeV, more preferably between 200 MeV and 250 MeV.

14. A particle therapy system according to claim 13, **characterized in that** that the minimal and maximal thickness and the diameter of first and the second energy attenuation members (10, 20) respectively lie in the intervals [1 mm, 100 mm] and [10 mm, 300 mm] and [10 mm, 300 mm], preferably these intervals are [1 mm, 50 mm] and [10 mm, 200 mm] and [10 mm, 200 mm], more preferably these intervals are [1 mm, 10 mm] and [10 mm, 100 mm] and [10 mm, 150 mm].

## Patentansprüche

1. Energieabschwächer (1) für die Abschwächung der Energie eines geladenen Partikelstrahls (2), der von einem Partikelbeschleuniger extrahiert ist, wobei der genannte Energieabschwächer (1) umfasst:
- ein erstes Energieabschwächungsorgan (10), das eine Strahleneingangsseite (11), die die Form eines Kreisrings einer Scheibe oder eines Abschnitts davon hat, und eine Strahlenausgangsseite (12), die die Form eines Teils einer ersten kontinuierlichen spiralförmigen Oberfläche aufweist, die eine erste Achse (A1) hat,
- ein zweites Energieabschwächungsorgan (20), das eine Strahleneingangsseite (21), die die Form eines Teils einer zweiten kontinuierlichen spiralförmigen Oberfläche hat, die eine zweite Achse (A2) hat, und eine Strahlenausgangsseite (22), die die Form eines Kreisrings oder einer Scheibe oder eines Abschnitts davon hat, aufweist,
wobei die erste Achse (A1) parallel zu oder mit der zweiten Achse (A2) zusammenfallend ist, wobei die erste und zweite spiralförmige Fläche einander gegenüberliegen und dieselbe Flankenrichtung haben und
- eine Antriebsgruppe, die entweder betriebsbereit an das erste und das zweite Energieabschwächungsorgan angeschlossen ist und zum Rotieren des ersten und des zweiten Energieabschwächungsorgans um jeweils die erste Achse (A1) und die zweite Achse (A2) konfiguriert ist oder betriebsbereit an das erste Energieabschwächungsorgan angeschlossen und zum Rotieren des ersten Energieabschwächungsorgans um die erste Achse (A1) konfiguriert ist, wobei das zweite Energieabschwächungsorgan fest ist.

2. Energieabschwächer (1) gemäß Anspruch 1, **dadurch gekennzeichnet, dass** die Strahleneingangsseite (11) des ersten Energieabschwächungsorgans (10) lotrecht zu der ersten Achse (A1) ist und darin, dass die Strahlenausgangsseite (22) des zweiten Energieabschwächungsorgans (20) lotrecht zu der zweiten Achse (A2) ist.

3. Energieabschwächer (1) gemäß Anspruch 1 oder 2, **dadurch gekennzeichnet, dass** die Antriebsgruppe einen ersten Motor (M1) zum Rotieren des ersten Energieabschwächungsorgans (10) um die erste Achse (A1) und einen zweiten Motor (M2) zum Rotieren des zweiten Energieabschwächungsorgans (20) um die zweite Achse (A2) umfasst.

4. Energieabschwächer (1) gemäß irgendeinem der voranstehenden Ansprüche, **dadurch gekennzeichnet, dass** die erste und die zweite spiralförmige Fläche zylindrische spiralförmige Flächen sind.

5. Energieabschwächer (1) gemäß Anspruch 4, **dadurch gekennzeichnet, dass** die erste und die zweite spiralförmige Fläche denselben Radius haben und dass die erste Achse (A1) dieselbe ist wie die zweite Achse (A2).

6. Energieabschwächer (1) gemäß Anspruch 5, **dadurch gekennzeichnet, dass** die erste und die zweite spiralförmige Fläche dieselbe Neigung haben.

7. Energieabschwächer (1) gemäß Anspruch 6, **dadurch gekennzeichnet, dass** die Antriebsgruppe geeignet ist, sich um das erste und / oder das zweite Energieabschwächungsorgan gemäß einer spiralförmigen Bewegung um die erste Achse (A1) zu bewegen.

8. Energieabschwächer (1) gemäß Anspruch 6 oder 7, **dadurch gekennzeichnet, dass** das erste und das zweite Energieabschwächungsorgan in Form und Größe identisch sind.

9. Energieabschwächer (1) gemäß Anspruch 4, **dadurch gekennzeichnet, dass** der Radius (R1) der ersten spiralförmigen Fläche kleiner ist als der Radius (R2) der zweiten spiralförmigen Fläche, dass die Neigung der ersten spiralförmigen Fläche kleiner ist als die Neigung der zweiten spiralförmigen Fläche und dass die erste Achse (A1) unterschiedlich von und parallel zu der zweiten Achse (A2) ist.

10. Energieabschwächer (1) gemäß irgendeinem der voranstehenden Ansprüche, **dadurch gekennzeichnet, dass** das erste Energieabschwächungsorgan (10) und / oder das zweite Energieabschwächungsorgan (20) aus Beryllium oder Kohlenstoffgraphit hergestellt sind.

11. Partikeltherapiesystem, umfassend einen Partikelbeschleuniger (100) und einen Energieabschwächer (1) gemäß irgendeinem der voranstehenden Ansprüche, wobei der genannte Energieabschwächer (1) in Bezug auf einen Partikelstrahl (2), der aus dem Partikelbeschleuniger (100) derart positioniert und ausgerichtet ist, dass der extrahierte Partikelstrahl (2) in den Energieabschwächer (1) an der Strahleneingangsseite (11) des ersten Energieabschwächungsorgans (10) eintritt und derart, dass der genannte extrahierte Partikelstrahl (2) aus dem Energieabschwächer (1) an der Strahlenausgangsseite (22) des zweiten Energieabschwächungsorgans (20) austritt.

12. Partikeltherapiesystem gemäß Anspruch 11, **dadurch gekennzeichnet, dass** der Partikelbeschleuniger (100) ein fester Energiebeschleuniger, bevorzugt ein Zyklotron, weiter bevorzugt ein Synchrozyklotron ist.

13. Partikeltherapiesystem gemäß Anspruch 11 oder 12, **dadurch gekennzeichnet, dass** der Partikelbeschleuniger (100) konfiguriert ist, um an seiner Ausgabe (110) einen Partikelstrahl (2) auszugeben, dessen maximale Energie zwischen 1 MeV und 500 MeV, bevorzugt zwischen 100 MeV und 300 MeV, weiter bevorzugt zwischen 200 MeV und 250 MeV inbegriffen ist.

14. Partikeltherapiesystem gemäß Anspruch 13, **dadurch gekennzeichnet, dass** die minimale und die maximale Dicke und der Durchmesser der ersten und der zweiten Energieabschwächungsorgane (10, 20) jeweils in den Intervallen [1 mm, 100 mm] und [10 mm, 300 mm] und [10 mm, 300 mm] liegen, bevorzugt betragen diese Intervalle [1 mm, 50 mm] und [10 mm, 200 mm] und [10 mm, 200 mm], weiter bevorzugt betragen diese Intervalle [1 mm, 10 mm] und [10 mm, 100 mm] und [10 mm, 150 mm].

## Revendications

1. Dégradeur d'énergie (1) pour atténuer l'énergie d'un faisceau de particules chargées (2) extrait d'un accélérateur de particules, ledit dégradeur d'énergie (1) comprenant :
- un premier élément d'atténuation d'énergie (10) présentant une face d'entrée de faisceau (11) ayant la forme d'un anneau ou d'un disque, ou bien d'une partie de celui-ci, et une face de sortie de faisceau (12) ayant la forme d'une partie d'une première surface hélicoïdale continue ayant un premier axe (A1),
- un second élément d'atténuation d'énergie (20) présentant une face d'entrée de faisceau (21) ayant la forme d'une partie d'une seconde surface hélicoïdale continue ayant un second axe (A2), et présentant une face de sortie de faisceau (22) ayant la forme d'un anneau ou d'un disque, ou bien d'une partie de celui-ci,
le premier axe (A1) étant parallèle au second axe (A2) ou coïncidant avec celui-ci, les première et seconde surfaces hélicoïdales se faisant face l'une l'autre et ayant la même chiralité, et
- un ensemble d'entraînement, soit fonctionnellement connecté au premier et au second élément d'atténuation d'énergie et configuré pour faire tourner les premier et second éléments d'atténuation d'énergie respectivement autour du premier axe (A1) et du second axe (A2), soit fonctionnellement connecté au premier élément d'atténuation d'énergie et configuré pour faire tourner le premier élément d'atténuation d'énergie autour du premier axe (A1), le second élément d'atténuation d'énergie étant fixe.

2. Dégradeur d'énergie (1) selon la revendication 1, **caractérisé en ce que** la face d'entrée de faisceau (11) du premier élément d'atténuation d'énergie (10) est perpendiculaire au premier axe (A1), et **en ce que** la face de sortie de faisceau (22) du second élément d'atténuation d'énergie (20) est perpendiculaire au second axe (A2).

3. Dégradeur d'énergie (1) selon la revendication 1 ou 2, **caractérisé en ce que** l'ensemble d'entraînement comprend un premier moteur (M1) pour faire tourner le premier élément d'atténuation d'énergie (10) autour du premier axe (A1) et un second moteur (M2) pour faire tourner le second élément d'atténuation d'énergie (20) autour du second axe (A2).

4. Dégradeur d'énergie (1) selon l'une quelconque des revendications précédentes, **caractérisé en ce que** les première et seconde surfaces hélicoïdales sont des surfaces hélicoïdales cylindriques.

5. Dégradeur d'énergie (1) selon la revendication 4, **caractérisé en ce que** les première et seconde surfaces hélicoïdales ont le même rayon, et **en ce que** le premier axe (A1) est le même que le second axe (A2).

6. Dégradeur d'énergie (1) selon la revendication 5, **caractérisé en ce que** les première et seconde surfaces hélicoïdales ont la même pente.

7. Dégradeur d'énergie (1) selon la revendication 6, **caractérisé en ce que** l'ensemble d'entraînement est adapté pour déplacer le premier et/ou le second élément d'atténuation d'énergie selon un mouvement hélicoïdal autour du premier axe (A1).

8. Dégradeur d'énergie (1) selon la revendication 6 ou 7, **caractérisé en ce que** les premier et second éléments d'atténuation d'énergie sont de forme et de taille identiques.

9. Dégradeur d'énergie (1) selon la revendication 4, **caractérisé en ce que** le rayon (R1) de la première surface hélicoïdale est plus petit que le rayon (R2) de la seconde surface hélicoïdale, **en ce que** la pente de la première surface hélicoïdale est inférieure à la pente de la seconde surface hélicoïdale, et **en ce que** le premier axe (A1) est différent du second axe (A2) et parallèle à celui-ci.

10. Dégradeur d'énergie (1) selon l'une quelconque des revendications précédentes, **caractérisé en ce que** le premier élément d'atténuation d'énergie (10) et/ou le second élément d'atténuation d'énergie (20) sont en béryllium ou en graphite de carbone.

11. Système de thérapie par particules comprenant un accélérateur de particules (100) et un dégradeur d'énergie (1) selon l'une quelconque des revendications précédentes, ledit dégradeur d'énergie (1) étant positionné et orienté par rapport à un faisceau de particules (2) extrait de l'accélérateur de particules (100), de telle manière que le faisceau de particules extrait (2) entre dans le dégradeur d'énergie (1) au niveau de la face d'entrée de faisceau (11) du premier élément d'atténuation d'énergie (10), et de manière telle que ledit faisceau de particules extrait (2) sorte du dégradeur d'énergie au niveau de la face de sortie de faisceau (22) du second élément d'atténuation d'énergie (20).

12. Système de thérapie par particules selon la revendication 11, **caractérisé en ce que** l'accélérateur de particules (100) est un accélérateur à énergie fixe, de préférence un cyclotron, de façon plus préférable un synchrocyclotron.

13. Système de thérapie par particules selon l'une des revendications 11 ou 12, **caractérisé en ce que** l'accélérateur de particules (100) est configuré pour fournir, au niveau de sa sortie (110), un faisceau de particules (2) dont l'énergie maximale est comprise entre 1 MeV et 500 MeV, de préférence entre 100 MeV et 300 MeV, de façon plus préférable entre 200 MeV et 250 MeV.

14. Système de thérapie par particules selon la revendication 13, **caractérisé en ce que** les épaisseurs minimale et maximale et le diamètre des premier et second éléments d'atténuation d'énergie (10, 20) se situent respectivement dans les intervalles compris entre [1 mm et 100 mm] et entre [10 mm et 300 mm] et entre [10 mm et 300 mm], de préférence ces intervalles sont compris entre [1 mm et 50 mm] et entre [10 mm et 200 mm] et entre [10 mm et 200 mm], de façon plus préférable ces intervalles sont compris entre [1 mm et 10 mm] et entre [10 mm et 100 mm] et entre [10 mm et 150 mm].
